Europäisches Patentamt

European Patent Office

Office européen des brevets

(11) Publication number: **0 415 638 A2**

# EUROPEAN PATENT APPLICATION

(21) Application number: **90309206.2**

(51) Int. Cl.5: **C12Q 1/68**

(22) Date of filing: **22.08.90**

(30) Priority: **22.08.89 JP 215490/89**

(43) Date of publication of application:
**06.03.91 Bulletin 91/10**

(84) Designated Contracting States:
**AT BE CH DE DK ES FR GB GR IT LI LU NL SE**

(71) Applicant: **SRL, INC.**
**4-1, Nishishinjuku 2-chome**
**Shinjuku-ku, Tokyo 163(JP)**

(72) Inventor: **Hikiji, Kazumasa**
**c/o Hachioji Lab. of SRL, Inc., 51 Komiya-cho**
**Hachioji-shi, Tokyo 192(JP)**

(74) Representative: **Harrison, David Christopher**
**et al**
**MEWBURN ELLIS 2 Cursitor Street**
**London EC4A 1BQ(GB)**

(54) **Methods for detecting variations in base sequences of nucleic acids and reagents therefor.**

(57) A method for detecting variations such as point mutations in base sequences of nucleic acids is disclosed. In the method, a mixed probe is hybridized with a sample nucleic acid on a carrier. The mixed probe includes a normal type probe having a base sequence complementary to normal base sequence and an abnormal type probe having a base sequence complementary to abnormal base sequence and one of said normal type probe and said abnormal type probe is labelled with a marker. The unbound probes are then washed off and the marker remaining on the carrier is then detected.

EP 0 415 638 A2

Xerox Copy Centre

# METHODS FOR DETECTING VARIATIONS IN BASE SEQUENCES OF NUCLEIC ACIDS AND REAGENTS THEREFOR

This invention relates to a method for detecting variations in base sequences of nucleic acids and to a reagent therefor.

Various useful substances are now being produced by the genetic engineering technique utilizing microorganisms as the hosts. The production is usually carried out by inserting a gene encoding a desired substance into an appropriate vector which can self-replicate in a host cell, transforming the host with the resulting recombinant vector, culturing the obtained transformant and recovering the desired substance from the culture. However, when the recombinant vector is replicated, it is possible that the gene encoding the desired substance, which is inserted in the vector, mutates so that the amino acid sequence encoded thereby changes. To eliminate this possibility, it is necessary to check whether the gene inserted in the vector mutated or not. Whether a gene mutated or not is also checked in diagnoses of hereditary diseases.

The mutation of a gene, especially the point mutation of a gene is conventionally checked as follows. First, a DNA region containing the mutated point is amplified by a gene amplification technique also known as polymerase chain reaction technique. On the other hand, a normal type probe having a base sequence complementary to the normal gene, and a abnormal type probe having a base sequence complementary to the abnormal gene containing the point-mutated site are separately prepared. The normal type and abnormal type probes are labelled with a marker such as a radio isotope marker, enzyme marker and a fluorescent marker. A sample gene or a portion of a gene amplified by the gene amplification technique is then fixed to a carrier or matrix such as nylon or nitrocellulose membrane. After blocking the non-specific sites on the carrier, one of the normal type and abnormal type probes is hybridized with the sample nucleic acid on the carrier. After washing, the marker remaining on the carrier is detected. The sample nucleic acid on the carrier is then denatured by alkaline treatment and is hybridized with another type probe, followed by washing and detection of the marker on the carrier. By comparing the amount of the marker detected on the carrier, whether the sample nucleic acid is normal or mutated is determined.

However, this conventional method requires very precise operations. Further, the results of the judgment are not always clear and it is often difficult to judge whether the sample nucleic acid is normal or mutated. The reason is as follows: For example, in the case where a point mutation in a gene is to be detected using probes consisting of 20 nucleotides (hereinafter indicated as "20-mer"), if the sample gene is normal type, the complementariness of the normal type probe with the sample gene is 20/20, that is, 100% complementary. On the other hand, when the abnormal type probe is hybridized with the normal sample gene, the complementariness is 19/20. The normal type probe with a complementariness of 20/20 has a higher affinity with the sample gene than the abnormal type probe with a complementariness of 19/20. Thus, by utilizing this difference in the affinity with the sample gene, washing is performed at a temperature at which the normal type probe is not washed off while the abnormal type probe is washed off. After the washing, whether the probe remains on the carrier or not is checked. If the sample gene is normal, the normal type probe remains hybridized with the sample gene on the carrier while the abnormal type probe is washed off. Thus, it can be determined that the sample gene is normal.

However, the difference in the affinity resulting from the difference between the complementariness of 20/20 and that of 19/20 is extremely small. Therefore, to carry out the test utilizing this extremely small difference in affinity, it is necessary to extremely precisely control the temperature of the washing solution, preferably in the order of 1/100 °C. However, it is very difficult to precisely control the temperature of the washing solution. As a result, the test results are not always clear and it is often difficult to determine whether the sample gene is normal or mutated, so that the reliability of the test is low. To promote the reliability of the test, the above-mentioned operations are repeated several times. Thus, in the conventional method, precise operation is required while its reliability is low in spite of the precise operation.

## SUMMARY OF THE INVENTION

Accordingly, an object of the present invention is to provide a method for detecting variations in base sequences of nucleic acids in which precise operations as required in the conventional method are not necessary and yet the test results are clear and reliable, as well as a reagent therefor.

The present inventor intensively studied to find that by carrying out the hybridization in a single step using a mixed probe containing a normal type probe and an abnormal type, probe, one of which is labelled, much clearer test results may be obtained even if the temperature of the washing solution is not precisely controlled. The present invention is based on this discovery.

That is, the present invention provides a method for detecting variations in base sequences of nucleic acids comprising the steps of hybridizing a mixed probe with a sample nucleic acid on a carrier, which mixed probe includes a normal type probe having a base sequence complementary to normal base sequence and an abnormal type probe having a base sequence complementary to abnormal base sequence, one of said normal type probe and said abnormal type probe being labelled with a marker; washing off unbound probes; and detecting said marker on said carrier.

In the method of the present invention, since the normal type and abnormal type probes simultaneously and competitively react with the sample nucleic acid, even if the difference in the affinity with the sample nucleic acid between the normal type and abnormal type probes is very small, the probe which has 100% complementariness with the sample nucleic acid wins the competition to hybridize with the sample nucleic acid. Therefore, by the method of the present invention, even if the precise temperature control during the washing step is not performed, clear and reliable results may be obtained. Thus, the method of the present invention gives more reliable results by simpler operations than the conventional method. The present invention also provides a reagent for carrying out the method of the present invention.

## BRIEF DESCRIPTION OF THE DRAWING

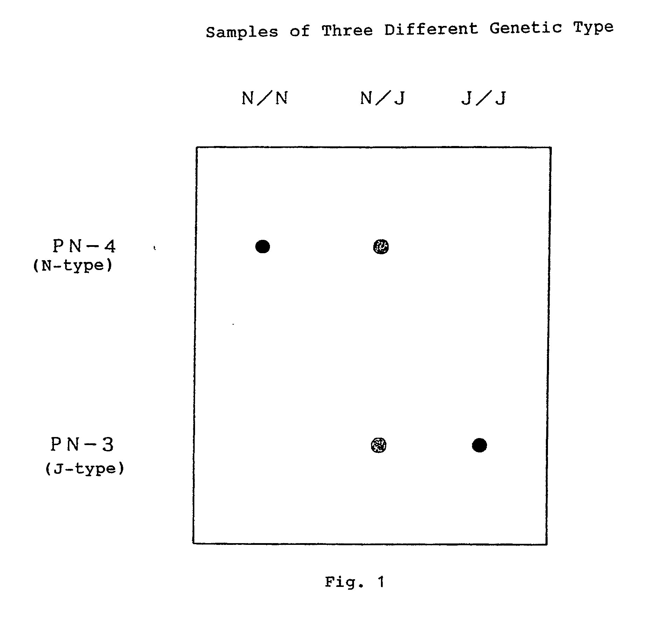

Fig. 1 is a schematic view showing test results obtained by the method of the present invention.

## DETAILED DESCRIPTION OF THE PREFERRED EMBODIMENTS

The sample nucleic acids which can be tested by the method of the present invention may be any nucleic acid, that is, any DNA and RNA, including DNAs such as genes and fragments thereof, DNAs other than genes such as promoters, and RNAs such as messenger RNAs.

Although nucleic acid separated from the body of an organism may be employed as the sample as it is, to improve the accuracy of the test, the sample nucleic acid may preferably be amplified by the gene amplification technique. Thus, in a preferred mode of the present invention, the sample is a nucleic acid amplified by the gene amplification technique. The gene amplification technique is known and is described by R. K. Saiki et al., Science, 230, 1350 (1985). In this technique, two nucleotide oligomers serving as primers, each of which is complementary with one of the both end portions of a DNA region to be amplified are prepared and the primers are hybridized with the DNA to be amplified. The DNA is then treated with *Taq* DNA polymerase so as to extend the DNA chains from the two primers. After stopping the extension of DNA chains, the resulting double-stranded DNA chains are denatured, and the two primers are again hybridized with the sample nucleic acid including the newly prepared progeny DNA chains, followed by stopping of the extension and subsequent denaturation. Thus, by repeating n times the cycle of extension of DNA chains, stopping of the extension and denaturation of the resulting DNA chains, the region to be amplified can be theoretically amplified $2^n$ times. Thus, by applying the gene amplification technique, even if the amount of the sample nucleic acid is very small, since it can be amplified as much as 1,000,000 times, the detection limit and the precision of the test may largely be promoted.

On the other hand, a normal type probe having a base sequence complementary with the normal type base sequence of the sample, and an abnormal type probe having a base sequence complementary with the abnormal base sequence are prepared. The probes may be chemically synthesized or may be prepared by amplifying a nucleic acid cut out from a natural source. One of the normal type probe and the abnormal type probe is labelled with a marker. The marker may be a conventional one such as an enzyme marker, fluorescent marker and a radioisotope marker. The mixing ratio of the labelled probe to the non-labelled probe is not restricted and may preferably be 1:1 to 1:5 in terms of molar ratio.

After blocking the non-specific binding sites on a carrier or matrix by a conventional method, the hybridization between the sample nucleic acid and the mixed probe is carried out on the carrier. The carrier or matrix may be a conventional one which has a large affinity with nucleic acids, such as a nitrocellulose membrane filter or a nylon filter. Since the sample nucleic acid fixed to the carrier must be a single-stranded chain, the sample nucleic acid is fixed to the carrier after converting it into single-stranded nucleic acid by alkali treatment or the like. The fixing of the single-stranded sample nucleic acid to the carrier may be carried out in a conventional manner by contacting a solution containing the single-stranded sample nucleic acid with the carrier.

The hybridization may be carried out by reacting an excess amount of the mixed probe with the sample nucleic acid on the carrier.

The hybridization may be carried out at a temperature in the vicinity of the $\alpha$ temperatures of the probes. The $\alpha$ temperature is the temperature at which the hybridization most well occurs, and it varies depending on the size of the probe and on the GC content of the probe. Probes having a length of about 20 nucleotides have an $\alpha$ temperature of $50°C$ - $60°C$. Probes larger than 20-mer normally have an $\alpha$ temperature within a range higher than this range and probes smaller than 20-mer normally have an a temperature within a range lower than this range.

In a preferred mode of the present invention, the hybridization is carried out while lowering the temperature from a temperature higher than the $\alpha$ temperatures of the normal type and abnormal type prod (since each probe has its $\alpha$ temperature, a temperature higher than the two $\alpha$ temperatures) to a temperature lower than the $\alpha$ temperatures of the probes. By carrying out the hybridization while lowering the temperature as such, the temperature of the reaction system necessarily passes the $\alpha$ temperatures of the probes. Thus, without precise control of the temperature, the hybridization may be well carried out, which is extremely advantageous. Although the $\alpha$ temperatures varies depending on the probes, the hybridization step may be well carried out by lowering the reaction temperature from $70°C$ to $50°C$.

In the above-described hybridization step, the normal type probe and the abnormal type probe competitively react with the sample nucleic acid. The probe with 100% complementariness wins the competition so as to hybridize with the sample nucleic acid while the other type of probe substantially cannot hybridize with the sample nucleic acid at all. This can always be attained by carrying out the reaction at a temperature in the vicinity of the $\alpha$ temperatures of the probes or by carrying out the reaction while lowering the temperature from a temperature higher than the $\alpha$ temperatures of the probes to a temperature lower than the $\alpha$ temperature of the probes as mentioned above.

After the hybridization, unbound probes, that is, the probe which does not have 100% complementariness and the excess probe having 100% complementariness are removed by washing. As mentioned above, since the sample nucleic acid is hybridized only with the probe with 100% complementariness, precise temperature control of the washing solution is not required and the washing can be performed simply aiming at removing the unbound probes. Thus, the operation is much easier than in the conventional method.

The marker which is used for labelling one of the normal type and abnormal type probes is then detected on the carrier. The detection of the marker may be carried out in a conventional manner. In the method of the present invention, since only one of the normal type and abnormal type probes is labelled with the marker, by detecting the marker on the carrier, it can be judged whether the sample is normal type or abnormal type.

In the method of the present invention, since only the probe with 100% complementariness hybridizes with the sample nucleic acid, the test results are very clear and so there is no difficulty in judging whether the sample nucleic acid is of normal type or abnormal type. Thus, the method of the present invention is highly reliable. Further, as described above, the method of the present invention does not require the precise temperature control as required in the conventional method.

The method of the present invention can be applied to any cases where it is necessary to check whether the base sequence of a DNA or RNA is normal or abnormal. For example, in cases where a desired protein is to be prepared by the genetic engineering technique, the method of the present invention may be employed for checking whether a gene encoding the desired protein, which is inserted in a vector, mutated or not as the recombinant vector replicates. The method of the present invention may also be employed for the diagnoses of hereditary diseases. Further, the method of the present invention can be applied for checking whether or not a chemically synthesized nucleic acid has a proper base sequence as designed.

Although the method of the present invention is most valuable when the affinity of the normal type and the abnormal type probes to the sample nucleic acid is almost the same, that is, when a point mutation of the sample nucleic acid is to be detected, the method of the present invention may also be applied for detectng the variations of the base sequence of the sample nucleic acid, in which 2 or more nucleotides are substituted. Further, the method of the present invention may be applied to the detection of variations of base sequences caused other than by mutation. For example, the method of the present invention can be applied for checking whether or not a chemically synthesized nucleic acid has a proper base sequence as designed, as mentioned above.

The present invention will now be described by way of examples thereof. It should be noted that the examples are presented for the illustration purpose only and should not be interpreted in any restrictive

4

way.

[Example]

Analysis of Mutation of APRT (Adenine Phosphoribosyl Transferase) Gene

(1) Extraction of DNAs

Lymphocytes were separated from 5 - 10 ml of human peripheral blood by specific gravity method and DNAs were extracted there from by a conventional method.

(2) Amplification of Specific Region by Gene Amplification Technique

The following oligonucleotides necessary for the gene amplification were synthesized by using a DNA synthesizer.

J-mutated region:

Primer 1, PN-10 (forward, 20-mer)

(2589) 5′-TGG AGC ACC TGC TCT CTG CA-3′ (2608)

Primer 1′: PN-5 (forward, 20-mer)

(2396) 5′-GAT GAT CTG CTG GCC ACT CG-3′ (2415)

Primer 2: PN-9 (reverse, 20-mer)

(2803) 5′-CAG CTG GAG ATG TTG GGC TG-3′ (2822)

(this is an antisense chain of GAG CCC AAC ATC TCC AGC TG-3′)

It should be noted that in the base sequences just described above, the numbers in parentheses indicate the base number of the APRT gene. The region to be amplified sandwiched between PN-10 and PN-9 has 234 bases and that sandwiched between PN-5 and PN-9 has 427 bases.

(3) Gene Amplification Method

(a) Preparation of Sample

An aliquote of the DNA sample obtained in (1) described above was taken and was diluted with Tris-EDTA (TE) buffer to a concentration of 0.1 μg/μl and to a volume of 50 μl. By heating the sample at 95°C for 5 minutes, the DNAs were denatured. After once stirred, the sample was centrifuged at 6000 rpm for 30 seconds and the resultant was used in the subsequent steps.

(b) Preparation of Reaction Mixture

The reaction mixtures having a composition described below per one tube were prepared.

| 10 x buffer: | 5.00 μl |
|---|---|
| dNTP mixture (4NTPs) | 8.00 μl |
| Primer 1 or 1′ (0.5 μg/μl) | 0.25 μl |
| Primer 2 (0.5 μg/μl) | 0.25 μl |
| Polymerase | 0.50 μl |
| Total | 14.00 μl |

(c) Gene Amplification

The above-described reaction mixture of 14.00 μl was mixed with 31.00 μl of distilled water and 5.00 μl of the DNA sample (0.1 μg/μl) obtained in (1) described above and the resulting mixture was centrifuged. Two drops of a mineral oil was added to the mixture and the mixture was again centrifuged. The tube was then set in an automatic temperature controller and a cycle of 94°C, 2 minutes-65°C, 3 minutes-72°C, 3 minutes was repeated 35 times so as to amplify the region sandwiched between the primers. The amplified sample was stored at 4°C.

4. Dot Blotting Method

(a) From 50 μl of the reaction mixture obtained in (3)(c), 40 μl aliquote was taken and was mixed with 400 μl of 0.4 M NaOH solution containing 25 mM EDTA•2Na.

(b) A filter paper and a Nylon filter (both had been immersed in 2 x SSC solution) were set in a dot blotting apparatus (Hybridot commercially available from Biorad Inc.), and 200 μl of each sample obtained in (c) was supplied to each well of the apparatus while aspirating the sample through the filter. After complete aspiration, 200 μl of 2 x SSC was supplied to each well so as to wash the filter.

(c) After removing the filters from the apparatus, they were irradiated with UV light for 2 minutes and subjected to the hybridization step hereinbelow described.

(5) Preparation of Probes and 5′-end Labelling Thereof

A normal type probe and an abnormal type (J-mutated) probe having the following base sequences, respectively, were chemically synthesized by a conventional method. Normal Type Probe, PN-4 (19-mer)

(2644) 5′-CAG GAA CCA TGA ACG CTG C-3′ (2662)

Abnormal Type (J-Mutated) Probe, PN-3 (19-mer)

(2644) 5′-CAG GAA CCA CGA ACG CTG C-3′ (2662)

A reaction mixture having the following composition was prepared and was incubated at 37°C for 1 hour. Thereafter, the reaction mixture was subjected to Quic Spin G25 column (commercially available from Boehringer-Mannheim) and the desired labelled probe was separated by centrifugation at 3000 rpm for 2 minutes.

| | |
|---|---|
| Probe | 0.6 µl |
| Distilled Water | 5.4 µl |
| Buffer C (commercially available from Boehringer) | 2.0 µl |
| $T_4$ Polynucleotide Kinase | 2.0 µl |
| $\gamma$-$^{32}$P-ATP | 10.0 µl |
| Total | 20.0 µl |

(6) Prehybridization and Hybridization

(a) Prehybridization

Twenty milliliters of a solution having the following composition was applied to a filter sizing 10 cm x 20 cm, and the resultant was incubated at 56°C for 2 hours.

5 x SSPE

5 x Denhaldt's Solution

0.5% SDS

(b) Hybridization To the filter prepared in (a), 4 - 5 ml of the labelled probe solution obtained above (2 - 5 x $10^6$ dpm/ml) containing 1 µg of antiprobe (in the case where the labelled probe is PN-3, the antiprobe is non-labelled PN-4, and in the case where the labelled probe is PN-4, the antiprobe is non-labelled PN-3) was applied. The filter was then incubated at 60°C for 30 minutes. The temperature was then lowered from 60°C to 50°C for 30 minutes and was incubated at 50°C for 30 minutes.

(7) Washing

The filter was washed with 2 x SSPE/0.1% SDS solution (i) twice at room temperature for 10 minutes and (ii) once at 50°C for 30 minutes.

(8) Autoradiography

(a) Autoradiography was performed for 3 - 12 hours by a conventional method.

(b) Results

The results are shown in Fig. 1.

It can be seen from Fig. 1, in the case where the genetic type of the sample APRT gene is N/N (normal/normal), when a mixed probe in which the normal type probe is labelled was used, a clear spot was observed and when a mixed probe in which the abnormal type (J-type) probe was used, no spot was detected. Similarly in the case where the genetic type of the sample APRT gene is J/J (J-type/J-type), when a mixed probe in which the normal type probe is labelled was used, no spot was detected and when a mixed probe in which the abnormal type (J-type) probe is labelled was used, a clear spot was observed. Further, in the case where the genetic type of the sample APRT gene was J/N, a spot having a lower density than the above-described clear was observed when either of the mixed probes was used.

From these results, it can be seen that by using the method of the present invention, extremely clear results may be obtained without precise temperature control.

Although the invention was described in detail by way of preferred examples thereof, it is apparent for those skilled in the art that various modifications may be made without departing from the spirit and scope of the present invention.

**Claims**

1. A method for detecting variations in base sequences of nucleic acids comprising the steps of:
hybridizing a mixed probe with a sample nucleic acid on a carrier, which mixed probe includes a normal type probe having a base sequence complementary to normal base sequence and an abnormal type probe having a base sequence complementary to abnormal base sequence, one of said normal type probe and said abnormal type probe being labelled with a marker;
washing off unbound probes; and
detecting said marker on said carrier.

2. The method of claim 1, wherein said hybridization is carried out while lowering the temperature during said hybridization from a temperature higher than α temperatures of said normal type and abnormal type probes to a temperature lower than said α temperatures of said normal type and abnormal type probes.

3. The method of claim 1, which is a method for detecting a point mutation.

4. The method of claim 2, which is a method for detecting a point mutation.

5. The method of claim 1, wherein said sample nucleic acid is one amplified by the gene amplification technique.

6. A reagent for detecting variations in base sequences of nucleic acids comprising a normal type probe having a base sequence complementary to normal base sequence and an abnormal type probe having a base sequence complementary to abnormal base sequence, one of said normal type probe and said abnormal type probe being labelled.

Samples of Three Different Genetic Type

N／N          N／J        J／J

P N−4
(N-type)

P N−3
(J-type)

Fig. 1